# EUROPEAN PATENT APPLICATION

(11) **EP 1 022 032 A2**
(43) Date of publication of application: **26.07.2000**
(21) Application number: 99125468.1
(22) Date of filing: 21.12.1999
(51) Int. Cl.: A61M 25/00

(54) **Percutaneous catheter for infusing drugs into a human body**

(30) Priority: 04.01.1999 IT MI990002
(71) Applicant: Medical Technology S.p.A., 22060 Novedrate (CO) (IT)
(72) Inventor: Greco, Francesco, c/o Medical Technology S.p.A., 22060 Novedrate (CO) (IT); Ricci, Stefano, c/o Medical Technology S.p.A., 22060 Novedrate (CO) (IT)
(74) Representative: Ferraiolo, Ruggero

(57) **Abstract**

The catheter (1) comprises two separate ducts in its interior and for its entire length, said ducts being a first duct (3) for the passage of the drug to be infused and a second duct (4) for the passage of a hardening fluid introduced from outside the catheter; a hole (5) through which the drug is to be released may be situated in any convenient longitudinal position in the first duct (3), while the second duct (4) is provided with an aperture (6) from which a mass of the hardening fluid may pass into the artery (15) to set the catheter in a desired position and permanently maintain it there.

## Description

The present invention concerns a percutaneous catheter for infusing drugs into a human body and, more particularly, a catheter capable of being connected to a container containing the drug (or mixture of such drugs) that have to be administered in such a manner as to enable the said drug or mixture of drugs to reach a given region of the human body through an afferent artery.

The technical field of the invention is that of the techniques employed in oncology and, more particularly, in the local and regional chemotherapies in which the drugs administered to the patient have to reach certain target organs or tissues in an aimed manner.

According to an opinion widely held among the operators of this sector, correct application of the aforesaid techniques requires one to ensure a continuous perfusion of the drugs at extremely low flow rates and within a clearly delimited area of the neoplastic organ or tissue.

The technique conventionally employed in this field provides for the use of containers to be inserted below the skin and connected to catheters that are then pushed into the artery afferent to the tumor area.

This type of treatment may be realized either surgically by isolating the arterial peduncle and inserting the cannule into it or, alternatively, percutaneously in accordance with the technique of angiographic catheterism and the use of recently acquired methods.

Nevertheless, the aforesaid surgical technique is usually associated with the eradication of the original tumor in the presence of hepatic metastases and does not readily lend itself for extensive application on account of the need for a complete surgical act in the truest sense of the term. The percutaneous technique, on the other hand, given its ease of use and the drastic reduction of the necessary hospitalization times, can be employed in day hospital conditions and therefore renders this therapeutic approach suitable for large-scale use.

The prior art is such as to comprise a single catheter conceived for percutaneous use and suitable for sending the drug to the are to be perfused, the catheter in question having been invented by the radiologist Dr. Aray. The known technique comprises the insertion of the catheter into the concerned artery, where it is made to slide along a wire ― generally made of steel ― that acts as a guide, the so-called Seldinger technique, the said wire being removed after the catheter has been pushed into the desired position.

The known technique is associated with some drawbacks, the principal one of which consists of the gradual displacement of the tip of the catheter from the desired application zone, so that it becomes difficult to keep the flow of the drug to the treatment area as constant and continuous as it should be. Furthermore, the known technique calls for a great deal of preliminary investigations and therefore the use of numerous and costly items of medical and surgical equipment.

The present invention overcomes the aforesaid drawbacks and offers advantages that will be explained further on in the present description; the invention is a percutaneous catheter that at its distal end is provided with a first hole to permit the passage of a guide wire for introducing the catheter into the concerned artery and, as will be specified in greater detail in the claims, comprises two separate ducts in its interior and for its entire length, a first duct for the passage of the drug and a second duct for the passage of a hardening fluid, the said first hole being situated at the distal end of the first duct while the second duct is provided with an aperture from which a mass of the hardening fluid may pass into the artery to set the catheter in a desired position and permanently maintain it there; a second hole may be placed in a convenient longitudinal position in the first duct to permit the drug to issue into the artery.

For the sake of simplicity in the specification, the said ducts for the drug and the hardening fluid will hereinafter be referred to, respectively, as the primary duct and the secondary duct. The said hardening fluid could be a tissue adhesive, for example, an acrylic-type surgical glue suitable for the purpose and capable of setting on the tissue of the artery when it hardens to block the catheter in a given position or a fluid that expands outside the catheter contained by a deformable membrane and permanently deforms the said membrane when it hardens to prevent the catheter becoming displaced from the desired position.

In a first embodiment the aperture of the secondary duct that permits the outflow of the surgical glue is situated downstream of the hole from which there issues the drug itself On issuing from its aperture, the glue forms an appropriate glue clot around the point from which it is released that will set on and adhere to the tissue of the artery. In a second embodiment, the aperture in the secondary duct providing the passage for the hardening fluid is sealed by a deformable membrane that has its perimeter welded to the perimeter of the said aperture, with the said membrane capable of dilating under the pressure of the hardening fluid somewhat in the manner of a balloon that expands until it completely occupies the cross-section area of the blood vessel and thus prevents the catheter from sliding within the said vessel.

In a third embodiment the aforesaid deformable membrane expands only to the extent necessary to occupy a part of the cross-section area of the blood vessel and, pressing against the wall of the vessel, prevents the catheter from becoming displaced and thus offering the additional advantage of letting the blood flow at the sides of the balloon.

The principal advantage of the invention lies in the fact that it prevents the catheter from sliding within the artery after it has been introduced and pushed into the desired position, maintaining it permanently in the said position, from which it can then perfuse the medicinal drug even for long periods of time, thus making it possible to convey a clearly defined quantity of the drug to the appropriate area of an organ or a tissue of the human body with the necessary continuity.

The invention will now be described in greater detail by means of some embodiment examples and reference to the attached drawings, where:
Figure 1 is a first perspective view,
Figure 2 is a section along the line A-A of Figure 1,
Figure 3 is a first partial section view,
Figure 4 is a second partial section view,
Figure 5 is a section along the line A-A of Figure 4,
Figure 6 is a third partial section view,
Figure 7 is a section along the line A-A of Figure 6, and
Figure 8 is a second perspective view.

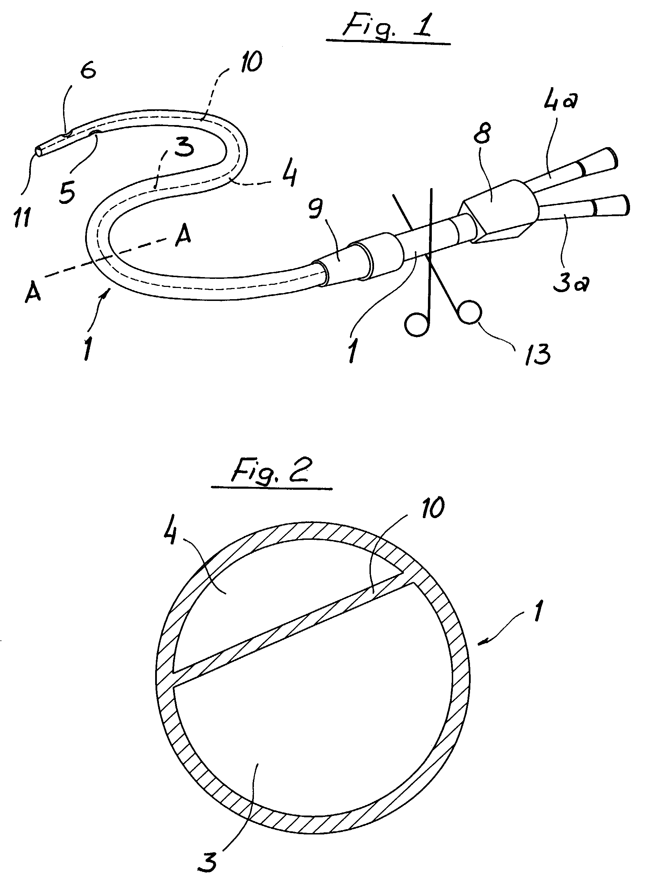

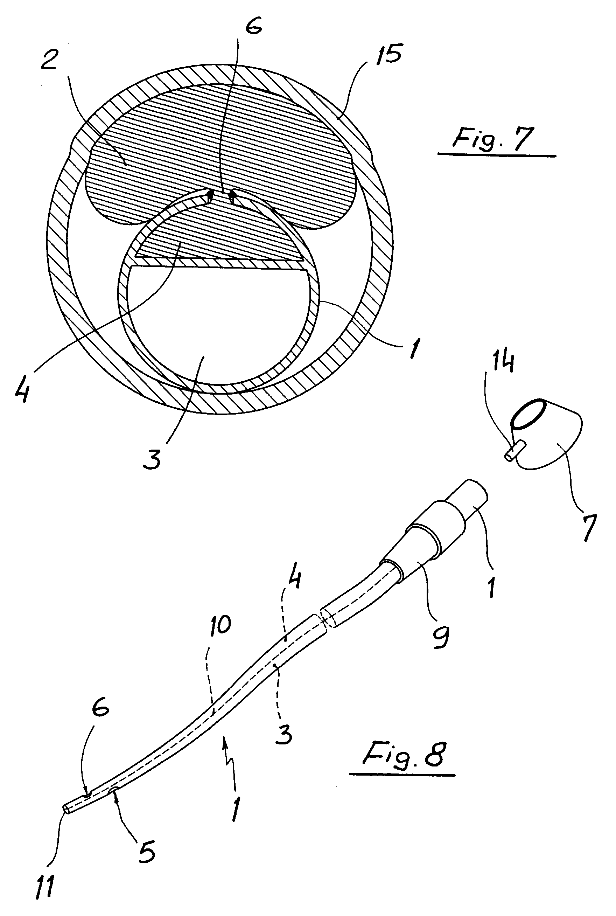

Figures 1 to 3 show a catheter 1 in which a longitudinal baffle 10 separates a primary duct 3 from a secondary duct 4 that has a smaller section than the primary duct and conveys a surgical glue capable of setting on and adhering to the tissue of an artery. The surgical glue employed in this embodiment is marketed under the name of GLUBRAN and is distributed by GEM s.r.l. of Viareggio, Italy. The figures also show the terminal hole 11 needed in order to enable the catheter to be slid into the blood vessel along a steel guide wire in accordance with the known Seldinger technique, the said wire being removed after the catheter has been placed in the desired position, the aperture 6 in the secondary duct for releasing the surgical glue and the lateral hole 5 in the primary duct 3 through which the medicinal drug passes into the artery 15 . As can clearly be seen, the aperture 6 is very close to the hole 11 , while the lateral hole 5 is situated upstream of the said hole 11 and opening 6 and in a position such that the said hole 5 will not become obstructed by the glue 12 and that it will find itself in a predefined position from which the drug is to be released. In an operative phase, following the injection of glue by means of a suitable syringe, the glue 12 is pushed out through the opening 6 and, since the said opening 6 is in the immediate vicinity of the terminal hole 11 , one also obtains the advantageous occlusion of the said terminal hole 11 . In this particular embodiment the catheter is also provided with a two-way connector 8 to connect the primary duct 3 and the secondary duct 4 to, respectively, the proximal connection 3a and 4a for the introduction of, respectively, the guide wire which is used in the first phase of positioning the catheter and then removed and the glue which is introduced after the catheter has been positioned. Also shown in the figure are the anchorage socket 9 and the scissors 13 , the use of which will be described in connection with Figure 7.

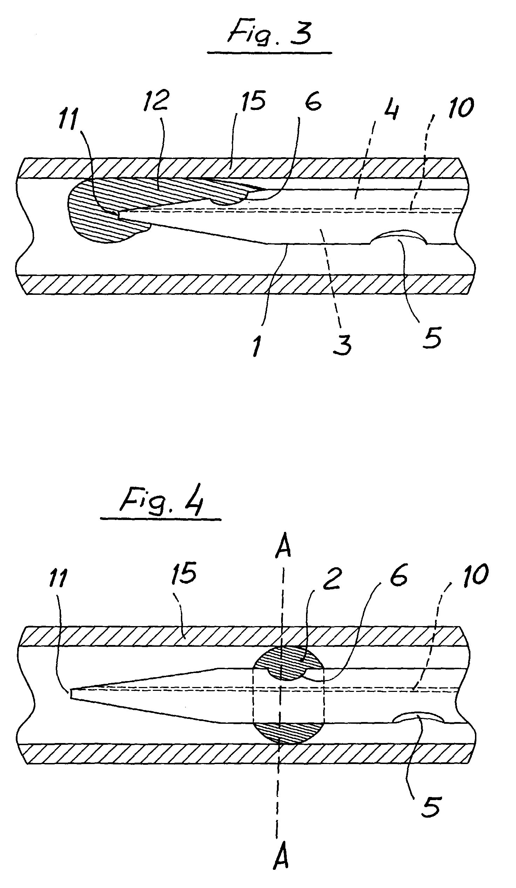

Figure 4 illustrates a second embodiment suitable for being applied in a terminal arteriole. In this embodiment the secondary duct of the catheter 1 is provided with an aperture 6 that is sealed by a deformable membrane 2 , the perimeter of the said deformable membrane being welded to the perimeter of the said aperture, the said deformable membrane being such as to contain the hardening fluid and to expand under the pressure of this fluid somewhat in the manner of a balloon. The catheter is also provided with a lateral hole 5 on the primary duct to permit the drug to be released into the artery. In this second embodiment the aperture 6 of the secondary duct 4 may be located in any convenient position and not necessarily at the distal end of the catheter in the immediate vicinity of the terminal hole 11 .

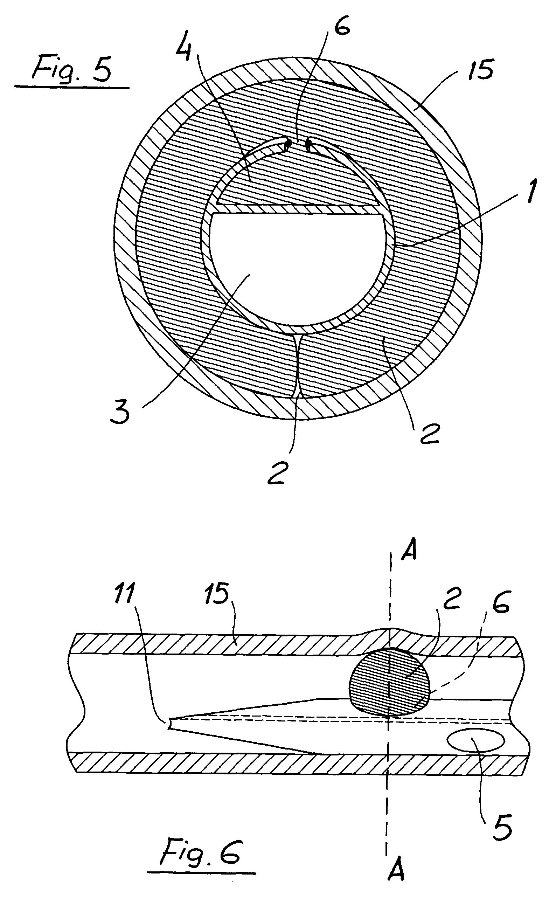

Figure 5 shows a deformable membrane 2 that is completely expanded in the form of a balloon outside the aperture 6 of the secondary duct 4 and completely occludes the cross section of an arteriole 15 .

Figures 6 and 7 relate to a third embodiment in which the expansion of the deformable membrane 2 is limited in extent and therefore causes only a partial blockage of the cross section of the arteriole, blood can therefore continue to flow past the sides of the balloon, which makes it possible for this embodiment of the catheter to be used also in arteries other than terminal ones. The figures show that the balloon has deformed the surface of the artery, thereby creating the engagement between the two parts that prevents the catheter from sliding within the artery. Figure 6 also shows the position of the hole 5 through which the drug is released.

Figure 8 shows the catheter 1 as positioned in an artery (which is not shown in the figure) and with the glue already introduced. With reference to Figure 1, the catheter has here been cut between the connector 8 and the anchorage socket 9 by means of the scissors 13 to permit the primary duct 3 to be connected, via the anchorage socket 9 , to the spout 14 of the container 7 containing the drug to be infused.

## Claims

1. A percutaneous catheter (1) for infusing drugs into a human body provided at its distal end with a hole (11) to permit the passage of a guide wire for the introduction of the catheter into the concerned artery, **characterized** in that it comprises two separate ducts in its interior and for its entire length, said two separate ducts being a first duct (3) for the passage of the drug and a second duct (4) for the passage of a hardening fluid, the said first hole (11) being situated at the distal end of the first duct (3), a second hole (5) through which the drug is released being located in a convenient longitudinal position in the first duct (3), the second duct (4) being provided with an aperture (6) from which a mass of the hardening fluid may pass into the artery to anchor the catheter in a desired position and permanently maintain it there.

2. A catheter in accordance with claim 1, characterized in that the hardening fluid that is released through the said aperture (6) is a glue (12) capable of setting on and adhering to the tissue of the artery.

3. A catheter in accordance with claim 1, characterized in that the hardening fluid that is released through the said aperture (6) expands within a deformable membrane (2) that will come to bear against the whole of the interior wall of the artery when the membrane dilates under the pressure of the hardening fluid.

4. A catheter in accordance with claim 1, characterized in that the hardening fluid expands from the said aperture (6) into a deformable membrane (2) that will come to bear only against a part of the interior wall of the artery when it dilates under the pressure of the hardening fluid.

5. A catheter in accordance with claim 1, characterized in that the said aperture (6) is situated downstream of the hole (5) through which the drug is released.

6. A catheter in accordance with claim 1, characterized in that it comprises a two-way connector (8) suitable for connecting the first duct (3) and the second duct (4) to the proximal connections (3a) and (4a) for permitting the introduction of, respectively, the guide wire and the hardening fluid.

7. A catheter in accordance with claim 6, characterized in that it comprises an anchorage socket (9) suitable for connecting the first duct (3) to the spout (14) of a drug containing container (7) once the catheter has been cut between the said two-way connector (8) and the anchorage socket (9).
